# EUROPEAN PATENT SPECIFICATION

(11) **EP 4 208 213 B1**
(45) Date of publication and mention of the grant of the patent: **10.06.2026**
(21) Application number: 20767748.5
(22) Date of filing: 01.09.2020
(51) Int. Cl.: A61L 27/12, A61L 27/22, A61L 27/56, A61L 27/58

(54) **METHOD FOR PRODUCING MACROPOROUS SCAFFOLDS COMPOSED OF SILK AND CALCIUM PHOSPHATE**
VERFAHREN ZUR HERSTELLUNG MAKROPORÖSER GERÜSTE AUS SEIDE UND CALCIUMPHOSPHAT
PROCÉDÉ DE PRODUCTION D'ÉCHAFAUDAGES MACROPOREUX COMPOSÉS DE SOIE ET DE PHOSPHATE DE CALCIUM

(43) Date of publication of application: 12.07.2023
(73) Proprietor: Spiber Technologies AB, 106 91 Stockholm (SE)
(72) Inventor: WIDHE, Mona, 753 21 Uppsala (SE); HEDHAMMAR, My, 113 51 Stockholm (SE); MESTRES, Gemma, 106 91 Stockholm (SE); DIEZ-ESCUDERO, Anna, 106 91 Stockholm (SE)
(74) Representative: AWA Sweden AB
(86) International application number: PCT/EP2020/074370
(87) International publication number: WO 2022/048733

(56) References cited:
- WO-A1-2016/100721
- CN-A- 109 260 511
- MORGAN ET AL: "Characterization and optimization of RGD-containing silk blends to support osteoblastic differentiation", BIOMATERIALS, ELSEVIER, AMSTERDAM, NL, vol. 29, no. 16, 5 March 2008 (2008-03-05), pages 2556 - 2563, XP022559053, ISSN: 0142-9612
- MCNAMARA STEPHANIE L ET AL: "Silk as a biocohesive sacrificial binder in the fabrication of hydroxyapatite load bearing scaffolds", BIOMATERIALS, ELSEVIER, AMSTERDAM, NL, vol. 35, no. 25, 29 May 2014 (2014-05-29), pages 6941 - 6953, XP028868109, ISSN: 0142-9612, DOI: 10.1016/J.BIOMATERIALS.2014.05.013

## Description

### Field of the invention

The present invention is concerned with bone regeneration, and more specifically with a method for producing macroporous scaffolds which are useful in bone regeneration. The macroporous scaffolds comprise silk and calcium phosphate.

### Background to the invention

Calcium phosphates (CaPs) are widely used for bone regeneration due to their resemblance to bone mineral phase. CaP is a family of mineral salts containing calcium and orthophosphate ions. They can be divided into two major categories depending on the processing temperature of the synthesis routes: high temperature CaP and low temperature CaP. High temperature CaP are produced by sintering at temperatures above 800 °C. They are similar in composition with the inorganic phase of calcified tissues and have been shown to be osteoconductive and bioresorbable. Alpha- and Beta-tricalcium phosphate (α-TCP and β-TCP) are two types of high temperature CaPs. Although the mechanical properties of CaPs can be improved upon sintering, nanostructures are lost, resulting from coalescence of the nanocrystals at high temperatures. Low temperature CaPs include amorphous calcium phosphate (ACP), octacalcium phosphate (OCP), hydroxyapatite (HA) or calcium deficient hydroxyapatite (CDHA), which are naturally occurring in the bones and highly relevant for bone graft substitutes.

A simple, straight-forward method for producing macroporous scaffolds may be foaming by mechanical stirring with addition of a small amount of surface-active molecules to the liquid phase, followed by mixing with the calcium phosphate powder. The liquid foam may be stabilized by incorporation of the powder, which reduces the gravitational drainage and air diffusion between the bubbles. The porous structures may be maintained through solidification of the cement. The foaming agents should meet the essential requirement of being water soluble and biocompatible in order to be used as the foaming template for macroporous calcium phosphate scaffolds (CPS). A low molecular weight, non-ionic surfactant, Polysorbate or namely Tween 80, is biocompatible and has been approved by the FDA as drug additives for parenteral administration. Macroporous CPSs have been successfully produced in previous studies using Tween 80 as the foaming agent, with interconnected pores and tunable pore size distribution by varying the surfactant concentration (Montufar, E. B., et al. Acta Biomaterialia 6.3 (2010), Díez Escudero, Anna, et al. Acta Biomaterialia 60 (2017), Barba, Albert, et al. ACS Applied Materials & Interfaces (2017):acsami.7b14175, Pastorino, David, C. Canal , and M. P. Ginebra Acta Biomaterialia 12 (2015)). The CPSs obtained were proved to be injectable, maintaining the macroporous structure after injection. High resorption rate was found to be promoted by incorporation of macroporosity during both *in vitro* and *in vivo* studies. Accelerated differentiation of rat mesenchymal stem cells (rMSCs) into osteoblasts was shown *in vitro,* resulting from the spherical macropores that offered the niches for osteogenic differentiation of the MSCs. Such macroporous CPSs foamed with Tween 80 have been studied as drug delivery systems, with tunable drug release profile through tailored macroporosity (Pastorino, David , C. Canal , and M. P. Ginebra, Acta Biomaterialia 12 (2015)). Another type of foaming agents which have been widely studied are amphiphilic proteins such as albumin and gelatin. These biomolecules may not only offer desirable foaming capacity, but also biological recognition *in vivo,* thereby promoting cell adhesion and bone ingrowth.

Polymers have also shown promising performance as three-dimensional matrices for bone regeneration purposes due to their biocompatibility, biodegradability and tunable properties. Polymers can be categorized as synthetic polymers such as polydioxanone (PDS), polycaprolactone (PCL), polyglycolic acid (PGA), polylactic acid (PLA), and poly (lactic-co-glycolic acid) (PLGA), and naturally derived polymers including chitosan, collagen and silk fibroin.

In spite of its highly desirable properties, a biopolymer alone is insufficient for repair of larger bone defects. Polymers lack compressive strength in regard of bone repair, and are more commonly used in composites with ceramics. Enhanced mechanical behavior and bone mimetic topography can be achieved by incorporation of mineral components, for instance bioceramics. Considerable attempts have been made to develop polymer-ceramic composites, taking advantage of the unique properties of the two materials to improve both the mechanical and biological behavior of scaffolds for bone tissue engineering. Polymer-ceramic composites are considered to be a promising candidate for bone graft substitute and regeneration applications. However, they lack interconnective porosity on the macro scale which plays a vital role in facilitating cell ingrowth, material associated-osteoinduction and replacement of the scaffold by newly-formed bone.

Silk fibroins are produced by numerous species of spiders and by worms from various insects such as bees, butterflies, and moths. Silks produced by silkworms (typically *Bombyx mori*) and orb-weaving spiders have desirable mechanical properties, environmental stability, biocompatibility, and tunable degradation.

WO 2014/066884 and McNamara et al, Biomaterials 35(25): 6941-6953 (2014) disclose ceramic materials comprising calcium phosphate material and silk, and methods for preparing the ceramic materials. The method comprises preparing a composition comprising silk and calcium phosphate (CaP), forming a green body from the composition and sintering at 1300°C or above of the green body to form the porous ceramic material.

WO 2016/100721 provides a silk ceramic material having enzymatically cross-linked amino acid side chains to generate injectable and flexible foam ceramics. Provided are compositions and methods of producing soft, flexible ceramic foam with silk polymeric crosslinking to serve as binders. The silk proteins are denatured by harsh solvents and require enzymatic formation of intermolecular covalent bonds to produce macrostructures.

Although various attempts have been made to introduce macroporosity to polymer-ceramic composites such as leaching, foaming, emulsion, replication, and rapid prototyping, there is still a great a need to be able to produce high strength, complex bio-active scaffolds, which are biocompatible, biodegradable, osteoconductive and even osteoinductive.

### Summary of the invention

It is an object of the present invention to provide a simplified method for producing a macroporous scaffold, suitable for use in bone tissue generation.

It is an object of the present invention to provide a method for producing a macroporous scaffold which does not require the use of non-biocompatible components.

For these and other objects that will be evident from the following description, the present invention provides according to a first aspect a method for producing a macroporous scaffold according to the appended claims.

### Brief description of the drawings

Fig 1 shows phase analyses of CPSs prepared with silk.
Fig 2 shows ATR-FTIR spectra of CPSs prepared with silk.
Fig 3 shows microscopic images of the CPSs prepared at different liquid/powder (L/P) ratios.
Fig 4 shows macroporosity of CPSs prepared with silk.
Fig 5 shows compressive strength of CPSs prepared with silk.
Fig 6 illustrates cell growth on CDHA with silk.
Fig 7 shows cross sections of Silk CDHA scaffolds after 3 weeks of cell culture.
Fig 8 shows cell viability of CDHA CPSs prepared with silk.
Fig 9 shows fluorescence micrographs of CPSs prepared with silk.
Fig. 10 illustrates the macroporosity of silk-foamed CPSs prepared with different L/P ratios.
Fig. 11 shows images of the macrostructures of CPSs prepared with silk.
Fig. 12 shows images of the macrostructures of CPSs prepared with silk and Tween respectively, with and without accelerator.

### List of appended sequences

### SEQ ID NO:

| | |
|---|---|
| 1 | non-functionalized spider silk ("4RepCT") |
| 2 | Z- functionalized spider silk ("Z-4RepCT") |
| 3 | FN_{cc}-functionalized spider silk ("FN_{cc}-4RepCT") |
| 4 | CT |
| 5 | CT *Aranaeus ventricosus* MiSp |
| 6 | IKVAV |
| 7 | YIGSR |
| 8 | EPDIM |
| 9 | NKDIL |
| 10 | GRKRK |
| 11 | KYGAASIKVAVSADR |
| 12 | NGEPRGDTYRAY |
| 13 | PQVTRGDVFTM |
| 14 | AVTGRGDSPASS |
| 15 | TGRGDSPA |
| 16 | CTGRGDSPAC |
| 17 | FN_{cc} motif |

### Detailed description of the invention

Calcium phosphate (CaP) is a family of mineral salts containing calcium and orthophosphate ions. They can be divided into two major categories depending on the processing temperature of the synthesis routes: high temperature CaP and low temperature CaP. High temperature CaP are produced by sintering at temperatures 800 °C. They are similar in composition with the inorganic phase of calcified tissues and have shown to be osteoconductive and bioresorbable. Alpha- and Beta-tricalcium phosphate (α-TCP and β-TCP) are two types of high temperature CaPs. Although the mechanical properties of CaPs can be improved upon sintering, nanostructures are lost, resulting from coalescence of the nanocrystals at high temperatures. Low temperature CaPs include amorphous calcium phosphate (ACP), octacalcium phosphate (OCP), hydroxyapatite (HA) or calcium deficient hydroxyapatite (CDHA), which are naturally occurring in the bones and highly relevant for bone graft substitutes

Calcium phosphate scaffolds (CPSs) are an unique type of low termperature CaP which have attracted great attention as bone graft substitute. CPSs may be obtained through mixing a CaP-containing powder with an aqueous phase, upon which a plastic paste is formed that can be injected and easily molded into various shapes.

Physicochemical, mechanical and biological behavior of the CPSs may be tailored by varying several factors concerning the manufacture process, such as additives, doping, liquid-to-powder (L/P) ratio and particle size of the starting materials.

Here, we design a facilitated method for producing macroporous scaffolds which are useful in bone regeneration. This method combines non-denatured silk protein with α-tricalcium phosphate (α-TCP) with a hydroxyapatite (HA) seed to produce macroporous scaffolds which are useful in bone regeneration.

The term "macroporous" refers to a porosity above 10 µm as it is considered to be generated from the foaming process, in contrast to the intrinsic microporosity (below 10 µm) of CPSs.

The macroporous scaffold is biocompatible and suitable for use in bone tissue generation. The resulting macroporosity is suitable for cell colonization and vascularization along gradual replacement of the implant by newly-formed bone. The production method is simple to perform and does not require the use of non-biocompatible components. Improved macroporosity is associated with enhanced vascularization, enhanced permeability, enhanced nutrient diffusion, and enhanced osteoinduction.

The term "biocompatible", as used herein, is defined as the ability of a material to perform with an acceptable host response in the body. It refers to components that do not cause significant harm to living tissue when placed in contact with such tissue, e.g., *in vivo.* Components are biocompatible if they are not toxic to cells. In additon, materials are biocompatible if their addition to cells *in vitro* results in less and/or no cell death, and/or their administration *in vivo* does not induce significant inflammation or other such adverse effects.

The present invention provides according to a first aspect an *in vitro* method for producing a macroporous scaffold, comprising the steps:
(a) subject a liquid phase which is an aqueous solution of a non-denatured silk protein to mechanical foaming, wherein the non-denatured silk protein is the foaming agent and wherein the non-denatured silk protein is capable of assembling into a water-insoluble macrostructure at a water/air interface;
(b) blend a mineral phase comprising α-tricalcium phosphate (α-TCP) with hydroxyapatite (HA) into the foamed liquid phase during continued mechanical foaming, thereby forming a macroporous paste;
(c) stabilize the macroporous paste into a desired shape;
(d) mineralize the stabilized, shaped porous paste into a macroporous calcium-deficient hydroxyapatite (CDHA)-based scaffold; wherein the method does not comprise any high-temperature sintering step; and wherein the method does not involve addition of detergents.

In the first step (a), a liquid phase which is an aqueous solution of a non-denatured silk protein is subjected to mechanical foaming.

For avoidance of doubt, foaming is the continuous formation of bubbles which have sufficiently high surface tension to remain as bubbles beyond the disengaging surface. Foaming may be used in various applications, such as industrial cleaning and preparation of building materials, and can be achieved through mechanical work (mechanical foaming) such as whipping, blending and stirring, but also through surface active components (surfactants) that reduce surface tension.

The foamability of the silk may arise from the alternating hydrophobic and hydrophilic sections in the peptide sequence, which allows it to adsorb to the air/water interface around the bubbles and stabilize them by reducing drainage and lamellae rupture. Silk concentration and foaming time may be two determining parameters for its foamability, since a foam can only be created when sufficient proteins are adsorbed to the interface within the foaming time scale. Adsorption may be enhanced by subsequent partial unfolding (surface denaturation) as a consequence of increased contact points at the interface. Increasing protein concentration may lead to higher amount of adsorption and better foaming properties.

The non-denatured silk protein dissolved in the aqueous solution is capable of assembling into a water-insoluble macrostructure at a water/air interface.

Importantly, the silk protein is present in soluble and non-denatured form in the liquid medium. This implies that silk protein is present in its native state, wherein it is properly folded, operative and functional. In contrast to the silk form used in the method disclosed herein, the presence of harsh solvents such as LiBr in the liquid medium denatures and solubilizes the silk protein.

The silk assembly process refers to when the silk proteins predominantly form bonds with the surrounding water molecules, irreversible formation of ordered polymers (foam) with predominantly intra- and intermolecular bonds between the silk proteins.

The assembly of the silk protein into foam is promoted by the presence of shearing forces and/or an interface between two different phases e.g. between a solid and a liquid phase, typically between air and a liquid phase or at a hydrophobic/hydrophilic interface, e.g. a mineral oil-water interface. The presence of the resulting interface may stimulate polymerization at the interface or in the region surrounding the interface, which region extends into the liquid medium, such that said polymerizing initiates at said interface or in said interface region.

Recombinantly produced silk is useful as matrices for culture of mammalian cells. The inclusion of cell adhesion motifs derived from the extracellular matrix (ECM) into such materials increases cell attachment and proliferation by interaction with integrins on the cell surface. The integrins do not just confer the physical connection between cells and the surrounding, but also mediate signals controlling for example cell growth, polarity, proliferation and survival. Moreover, the integrins are essential for cell migration by acting as the cells' "feet".

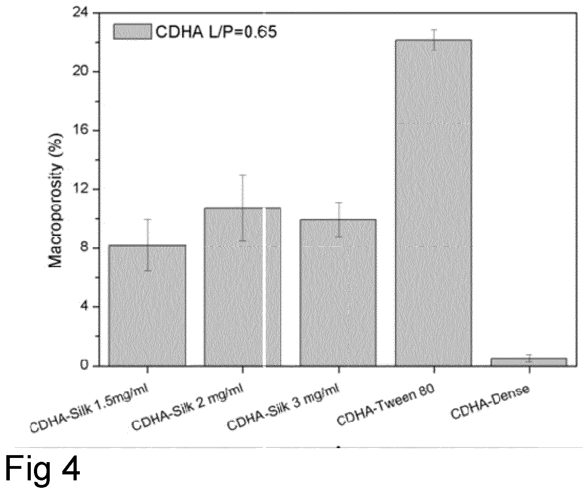

The silk protein is typically present in a concentration of 0.5 -5 mg/ml, preferably 2 mg/ml or higher, in the liquid phase. Increasing protein concentration should lead to higher amount of adsorption and better foaming properties, yet a threshold concentration of 2 mg/ml was observed (shown in Figure 4), above which the foamability of silk seemed to be "saturated".

The silk protein is typically foamed for short time periods, shorter than 30 s, preferably shorter than 10 s.The porosities shown in the results were all obtained using an optimal foaming time of 5 s. Prolonged foaming time led to increased small bubbles and less stable foams, which caused a decrease in macroporosity of the CPSs.

In a certain embodiment the method for producing a macroporous scaffold as disclosed herein the silk protein is a recombinant silk protein.

In a specific embodiment the method for producing a macroporous scaffold as disclosed herein the silk protein is a spider silk protein.

The terms "spidroins" and "spider silk proteins" are used interchangeably throughout the description and encompass all known spider silk proteins, including major ampullate spider silk proteins which typically are abbreviated "MaSp", or "ADF" in the case *of Araneus diadematus.* These major ampullate spider silk proteins are generally of two types, 1 and 2. These terms furthermore include non-natural proteins with a high degree of identity and/or similarity to the known spider silk proteins.

In some preferred embodiments of these and other aspects of the invention, the spider silk protein is comprising, or consisting of, the protein moieties **REP** and **CT,** wherein
**REP** is a repetitive fragment of from 70 to 300 amino acid residues, selected from the group consisting of **L(AG)ₙL, L(AG)ₙAL, L(GA)ₙL,** and **L(GA)ₙGL,** wherein **n** is an integer from 2 to 10; each individual **A** segment is an amino acid sequence of from 8 to 18 amino acid residues, wherein from 0 to 3 of the amino acid residues are not Ala, and the remaining amino acid residues are Ala; each individual **G** segment is an amino acid sequence of from 12 to 30 amino acid residues, wherein at least 40% of the amino acid residues are Gly; and each individual **L** segment is a linker amino acid sequence of from 0 to 30 amino acid residues; and
**CT** is a fragment of from 70 to 120 amino acid residues, having at least 70% identity to either of SEQ ID NO: 4-5.

Optionally, a functional moiety is arranged either terminally in the spider silk protein, or between the moieties, or within any of the moieties, preferably terminally in the spider silk protein.

The spider silk protein typically comprises from 140 to 2000 amino acid residues, such as from 140 to 1000 amino acid residues, such as from 140 to 600 amino acid residues, preferably from 140 to 500 amino acid residues, such as from 140 to 400 amino acid residues. The small size is advantageous because longer proteins containing spider silk protein fragments may form amorphous aggregates, which require use of harsh solvents for solubilisation and polymerisation.

The spider silk protein may contain one or more linker peptides, or L segments. The linker peptide(s) may be arranged between any moieties of the spider silk protein, e.g. between the **REP** and **CT** moieties, at either terminal end of the spider silk protein or between the spidroin fragment and the cell-binding motif. The linker(s) may provide a spacer between the functional units of the spider silk protein, but may also constitute a handle for identification and purification of the spider silk protein, e.g. a His and/or a Trx tag. If the spider silk protein contains two or more linker peptides for identification and purification of the spider silk protein, it is preferred that they are separated by a spacer sequence, e.g. His₆-spacer-His₆-. The linker may also constitute a signal peptide, such as a signal recognition particle, which directs the spider silk protein to the membrane and/or causes secretion of the spider silk protein from the host cell into the surrounding medium. The spider silk protein may also include a cleavage site in its amino acid sequence, which allows for cleavage and removal of the linker(s) and/or other relevant moieties. Various cleavage sites are known to the person skilled in the art, e.g. cleavage sites for chemical agents, such as CNBr after Met residues and hydroxylamine between Asn-Gly residues, cleavage sites for proteases, such as thrombin or protease 3C, and self-splicing sequences, such as intein self-splicing sequences.

The protein moiety **REP** is fragment with a repetitive character, alternating between alanine-rich stretches and glycine-rich stretches. The **REP** fragment generally contains more than 70, such as more than 140, and less than 300, preferably less than 240, such as less than 200, amino acid residues, and can itself be divided into several **L** (linker) segments, **A** (alanine-rich) segments and **G** (glycine-rich) segments, as will be explained in more detail below. Typically, said linker segments, which are optional, are located at the **REP** fragment terminals, while the remaining segments are in turn alanine-rich and glycine-rich. Thus, the **REP** fragment can generally have either of the following structures, wherein n is an integer:
**L**(**AG**)ₙ**L**, such as **LA₁G₁A₂G₂A₃G₃A₄G₄A₅G₅L;**
**L**(**AG**)ₙ**AL**, such as **LA₁G₁A₂G₂A₃G₃A₄G₄A₅G₅A₆L;**
**L**(**GA**)ₙ**L**, such as **LG₁A₁G₂A₂G₃A₃G₄A₄G₅A₅L;** or
**L**(**GA**)ₙ**GL**, such as **LG₁A₁G₂A₂G₃A₃G₄A₄G₅A₅G₆L.**

It follows that it is not critical whether an alanine-rich or a glycine-rich segment is adjacent to the N-terminal or C-terminal linker segments. It is preferred that n is an integer from 2 to 10, preferably from 2 to 8, also preferably from 4 to 8, more preferred from 4 to 6, i.e. n=4, n=5 or n=6.

In some embodiments, the alanine content of the **REP** fragment is above 20%, preferably above 25%, more preferably above 30%, and below 50%, preferably below 40%, more preferably below 35%. It is contemplated that a higher alanine content provides a stiffer and/or stronger and/or less extendible fiber.

In certain embodiments, the **REP** fragment is void of proline residues, i.e. there are no Pro residues in the **REP** fragment.

Turning now to the segments that constitute the **REP** fragment, it is emphasized that each segment is individual, i.e. any two **A** segments, any two **G** segments or any two **L** segments of a specific **REP** fragment may be identical or may not be identical. Thus, it is not a general feature of the spidroin that each type of segment is identical within a specific **REP** fragment. Rather, the following disclosure provides the skilled person with guidelines how to design individual segments and gather them into a **REP** fragment, which is a part of a functional spider silk protein useful in a cell scaffold material.

Each individual **A** segment is an amino acid sequence having from 8 to 18 amino acid residues. It is preferred that each individual **A** segment contains from 13 to 15 amino acid residues. It is also possible that a majority, or more than two, of the **A** segments contain from 13 to 15 amino acid residues, and that a minority, such as one or two, of the **A** segments contain from 8 to 18 amino acid residues, such as 8-12 or 16-18 amino acid residues. A vast majority of these amino acid residues are alanine residues. More specifically, from 0 to 3 of the amino acid residues are not alanine residues, and the remaining amino acid residues are alanine residues. Thus, all amino acid residues in each individual **A** segment are alanine residues, with no exception or with the exception of one, two or three amino acid residues, which can be any amino acid. It is preferred that the alanine-replacing amino acid(s) is (are) natural amino acids, preferably individually selected from the group of serine, glutamic acid, cysteine and glycine, more preferably serine. Of course, it is possible that one or more of the **A** segments are all-alanine segments, while the remaining **A** segments contain 1-3 non-alanine residues, such as serine, glutamic acid, cysteine or glycine.

In an embodiment, each **A** segment contains 13-15 amino acid residues, including 10-15 alanine residues and 0-3 non-alanine residues as described above. In a more preferred embodiment, each **A** segment contains 13-15 amino acid residues, including 12-15 alanine residues and 0-1 non-alanine residues as described above. Without wishing to be bound by any particular theory, it is envisaged that **A** segments according to the invention form helical structures or beta sheets.

Furthermore, it has been concluded from experimental data that each individual **G** segment is an amino acid sequence of from 12 to 30 amino acid residues. It is preferred that each individual **G** segment consists of from 14 to 23 amino acid residues. At least 40% of the amino acid residues of each **G** segment are glycine residues. Typically, the glycine content of each individual **G** segment is in the range of 40-60%.

In certain embodiments, the first two amino acid residues of each **G** segment are not -Gln-Gln-.

Each individual **L** segment represents an optional linker amino acid sequence, which may contain from 0 to 30 amino acid residues, such as from 0 to 20 amino acid residues. While this segment is optional and not critical for the function of the spider silk protein, its presence still allows for fully functional spider silk proteins and polymers thereof which form fibers, films, foams and other structures. In particular, the amino acid sequence of a linker segment may resemble any of the described **A** or **G** segments, but usually not sufficiently to meet their criteria as defined herein.

In one embodiment of the **REP** fragment, one of the **L** segments contains 0 amino acids, i.e. one of the **L** segments is void. In another embodiment of the **REP** fragment, both **L** segments contain 0 amino acids, i.e. both **L** segments are void. Thus, these embodiments of the **REP** fragments according to the invention may be schematically represented as follows: **(AG)ₙL, (AG)ₙAL, (GA)ₙL, (GA)ₙGL; L(AG)ₙ, L(AG)ₙA, L(GA)ₙ, L(GA)ₙG;** and **(AG)ₙ, (AG)ₙA, (GA)ₙ, (GA)ₙG.** Any of these **REP** fragments are suitable for use with any **CT** fragment as defined below.

The **CT** fragment of the spidroin in the cell scaffold material has a high degree of similarity to the C-terminal amino acid sequence of spider silk proteins. As shown in WO 2007/078239, this amino acid sequence is well conserved among various species and spider silk proteins, including MaSp1, MaSp2 and MiSp (minor ampullate spidroin).

It is not critical which specific **CT** fragment is present in the spider silk protein in the cell scaffold material. A representative **CT** fragment is the amino acid residues 166-263 of SEQ ID NO: 1 derived from *Euprosthenops australis,* i.e. SEQ ID NO. 4. Another representative **CT** fragment is the MiSp sequence SEQ ID NO: 5. Thus, in one embodiment, the **CT** fragment has at least 70%, such as at least 80%, such as at least 85%, preferably at least 90%, such as at least 95%, identity to SEQ ID NO: 4 or SEQ ID NO: 5. The **CT** fragment may be identical to SEQ ID NO: 4 or SEQ ID NO: 5

The **CT** fragment typically consists of from 70 to 120 amino acid residues. It is preferred that the **CT** fragment contains at least 70, or more than 80, preferably more than 90, amino acid residues. It is also preferred that the **CT** fragment contains at most 120, or less than 110 amino acid residues. A typical **CT** fragment contains approximately 100 amino acid residues.

The term "% identity", as used herein, is calculated as follows. The query sequence is aligned to the target sequence using the CLUSTAL W algorithm (Thompson et al, Nucleic Acids Research, 22:4673-4680 (1994)). A comparison is made over the window corresponding to the shortest of the aligned sequences. The amino acid residues at each position are compared, and the percentage of positions in the query sequence that have identical correspondences in the target sequence is reported as % identity.

The term "% similarity", as used herein, is calculated as described above for "% identity", with the exception that the hydrophobic residues Ala, Val, Phe, Pro, Leu, Ile, Trp, Met and Cys are similar; the basic residues Lys, Arg and His are similar; the acidic residues Glu and Asp are similar; and the hydrophilic, uncharged residues Gln, Asn, Ser, Thr and Tyr are similar. The remaining natural amino acid Gly is not similar to any other amino acid in this context.

Throughout this description, alternative embodiments according to the invention fulfill, instead of the specified percentage of identity, the corresponding percentage of similarity. Other alternative embodiments fulfill the specified percentage of identity as well as another, higher percentage of similarity, selected from the group of preferred percentages of identity for each sequence. For example, a sequence may be 70% similar to another sequence; or it may be 70% identical to another sequence; or it may be 70% identical and 90% similar to another sequence.

In a preferred spider silk protein according to the invention, the **REP-CT** fragment has at least 70%, such as at least 80%, such as at least 85%, preferably at least 90%, such as at least 95% or even 100% identity to SEQ ID NO: 1 or SEQ ID NO: 3.

The silk proteins are advantageously further containing a functional moiety. This functional moiety may be protein moiety as exemplified herein or a non-protein moiety. The functional moiety is arranged either terminally in the silk protein or within the silk protein, preferably N-terminally or C-terminally in the silk protein.

Desired bioactivities may be introduced by various bioactive proteins and peptides, such as a fibronectin peptide motif, which enhances cell adhesion and proliferation on the silk coatings, and the antimicrobial peptide Magainin I. The potential to include functional moieties is crucial in biomaterial applications in order to optimize the acceptance of the implants in the body and to tackle infection issues, which are also challenging successful implantation. Furthermore, more advanced bioactivities can be introduced using silk proteins fused to functional moieties in the form of protein domains with fold-dependent functions, such as affinity domains (e.g. Z domain binding IgG), enzymes (e.g. xylanase) or growth factors (e.g. fibroblast growth factor, FGF).

The spidroin fragment and the functional moiety are linked directly or indirectly to one another. A direct linkage implies a direct covalent binding between the moieties without intervening sequences, such as linkers. An indirect linkage also implies that the moieties are linked by covalent bonds, but that there are intervening sequences, such as linkers and/or one or more further moieties, e.g. 1-2 NT moieties.

The functional moiety may be arranged internally or at either end of the spider silk protein, i.e. C-terminally arranged or N-terminally arranged. It is preferred that the functional moiety is arranged at the N-terminal end of the spider silk protein. If the spider silk protein contains one or more linker peptide(s) for identification and purification of the spider silk protein, e.g. a His or Trx tag(s), it is preferred that it is arranged at the N-terminal end of the spider silk protein.

A preferred spider silk protein has the form of an N-terminally arranged functional moiety, coupled by a linker peptide of 0-30 amino acid residues, such as 0-10 amino acid residues, to a **REP** moiety. Optionally, the spider silk protein has an N-terminal or C-terminal linker peptide, which may contain a purification tag, such as a His tag, and a cleavage site.

The silk protein optionally contains a cell-binding motif (CBM) as a functional moiety. The optional cell-binding motif is arranged either terminally in the silk protein or within the silk protein, preferably N-terminally or C-terminally in the silk protein.

In certain preferred embodiments of these and other aspects of the invention, the functional moiety is a cell-binding motif selected from RGD, IKVAV (SEQ ID NO: 6), YIGSR (SEQ ID NO: 7), EPDIM (SEQ ID NO: 8), NKDIL (SEQ ID NO: 9), GRKRK (SEQ ID NO: 10), KYGAASIKVAVSADR (SEQ ID NO: 11), NGEPRGDTYRAY (SEQ ID NO: 12), PQVTRGDVFTM (SEQ ID NO: 13), AVTGRGDSPASS (SEQ ID NO: 14), TGRGDSPA (SEQ ID NO: 15), CTGRGDSPAC (SEQ ID NO: 16) and FN_{cc} (SEQ ID NO: 17); and preferably from FN_{cc}, GRKRK, IKVAV, RGD and CTGRGDSPAC, more preferably FN_{cc} and CTGRGDSPAC; wherein FN_{cc} is C¹X¹X²RGDX³X⁴X⁵C²; wherein each of X¹, X², X³, X⁴ and X⁵ are independently selected from natural amino acid residues other than cysteine; and C¹ and C² are connected via a disulphide bond.

In its most general form, FN_{cc} is C¹X¹X²RGDX³X⁴X⁵C² (SEQ ID NO: 17); wherein each of X¹, X², X³, X⁴ and X⁵ are independently selected from natural amino acid residues other than cysteine; and C¹ and C² are connected via a disulphide bond. FN_{cc} is a modified cell-binding motif that imitates the α5β1-specific RGD loop motif of fibronectin by positioning cysteines in precise positions adjacent to the RGD sequence to allow formation of a disulphide-bridge to constrain the chain into a similar type of turn loop. This cyclic RGD cell-binding motif increases the cell adhesion efficacy to a matrix made of a protein containing the cell-binding motif, such as a recombinantly produced spider silk protein. The term "cyclic" as used herein refers to a peptide wherein two amino acid residues are covalently bonded via their side chains, more specifically through a disulfide bond between two cysteine residues. The cyclic RGD cell-binding motif FN_{cc} promotes both proliferation of and migration by primary cells. Human primary cells cultured on a cell scaffold material containing the cyclic RGD cell-binding motif show increased attachment, spreading, stress fiber formation and focal adhesions compared to the same material containing a linear RGD peptide.

In preferred embodiments of FN_{cc}, each of X¹, X², X³, X⁴ and X⁵ are independently selected from the group of amino acid residues consisting of: G, A, V, S, T, D, E, M, P, N and Q. In other preferred embodiments of FN_{cc}, each of X¹ and X³ are independently selected from the group of amino acid residues consisting of: G, S, T, M, N and Q; and each of X², X⁴ and X⁵ are independently selected from the group of amino acid residues consisting of: G, A, V, S, T, P, N and Q. In certain preferred embodiments of FN_{cc}, X¹ is selected from the group of amino acid residues consisting of: G, S, T, N and Q; X³ is selected from the group of amino acid residues consisting of: S, T and Q; and each of X², X⁴ and X⁵ are independently selected from the group of amino acid residues consisting of: G, A, V, S, T, P and N. In some preferred embodiments of FN_{cc}, X¹ is S or T; X² is G, A or V; preferably G or A; more preferably G; X³ is S or T; preferably S; X⁴ is G, A, V or P; preferably G or P; more preferably P; and X⁵ is G, A or V; preferably G or A; more preferably A.

In certain preferred embodiments of FN_{cc}, the cell-binding motif is comprising the amino acid sequence CTGRGDSPAC (SEQ ID NO: 16). Further preferred cyclic RGD cell-binding motifs according to the invention display at least 60%, such as at least 70%, such as at least 80%, such as at least 90% identity to CTGRGDSPAC (SEQ ID NO: 16), with the proviso that position 1 and 10 are always C; position 4 is always R; position 5 is always G; position 6 is always D; and positions 2-3 and 7-9 are never cysteine. It is understood that the non-identical positions among positions 2-3 and 7-9 can be freely selected as set out above.

A preferred group of cell-binding motifs are FN_{cc}, GRKRK, IKVAV, and RGD, and in particular FN_{cc}, such as CTGRGDSPAC.

A preferred silk protein with an FN_{cc} cell-binding motif has the amino acid sequence of SEQ ID NO: 3.

In the subsequent step (b), a mineral phase comprising α-tricalcium phosphate (α-TCP) with hydroxyapatite (HA) is blended into the foamed liquid phase from step (a) during continued mechanical foaming, thereby forming a macroporous paste;

It has surprisingly been found that the silk protein holds the potential as a foaming agent to produce macroporous CPSs for bone tissue engineering and bone regeneration. Silk-incorporated CPSs showed enhanced cohesion properties and silk-based CPS showed no negative effect on the final composition. Silk-foamed CPSs showed cohesiveness after only 2 h, which was faster than the specimens prepared with Tween 80 solution regardless of the consolidation process. Furthermore, silk was shown to have the potential to be used as a foaming template for macroporous CPSs, with tunable porosity by varing the concentration of silk. This is particularly surprising in view of the various methods that have been developed.

When combined with hydroxyapatite, the carbonyl groups (>C=O) in the peptide bonds of silk can serve as the crystal nucleation sites, favoring anisotropic crystal growth. Microstructures revealed by SEM showed a mixture of needle and plate-like crystals in the silk-foamed CPSs, while only plate-like structures were obtained in the specimens foamed with Tween 80, indicating that a preferential orientation of crystal growth was facilitated by silk. Moreover, a more compact structure was observed for silk-foamed CPSs, as small crystals tend to be tightly arranged. The underlying mechanism has been explained in previous studies as the calcium ions chelated by the carbonyl oxygens can further interact with the phosphate groups, allowing continuous growth of hydroxyapatite crystals along the c-axis. Consequently, higher nanoporosity and specific surface area are generated, which are critical to material-associated osteoinduction and bioresorbability *in vivo.*

A hydroxyapatite seed is present in the mineral phase to provide nucleation sites for hydroxyapatite crystals. The initial hydroxyapatite concentration in the mineral phase is typically 0.1 - 10 wt%, such as 1 - 5 wt%, e.g. about 2 wt%.

Up to present, two types of CPSs are commercially available: hydroxyapatite (HA, Ca₉(HPO₄)(PO₄)₅(OH), most stable above pH 4.2) and brushite (CaHPO₄·2H₂O, stable under pH 4.2). Apatite cements are considered to be relevant for bone regeneration due to that the inorganic phase of natural bones comprises substituents-containing HA. Biomimetic HA are typically calcium-deficient HA (CDHA, Ca₁₀₋ₓ(HPO₄)ₓ(PO₄)₆₋ₓ(OH)₂₋ₓ). HA or CDHA are stable under physiological condition and commonly prepared through hydrolysis of α-tricalcium phosphate (α-TCP):

3α-Ca₃(PO₄)₂+H₂O→Ca₉(HPO₄)(PO₄)₅(OH)

The incorporation of silk altered the nucleation and crystal growth mechanisms of the CPSs, as minor needle- and plate-like crystals were found on silk samples, compared to Tween dense and porous samples.

This aspect of the invention is advantageous in that the foaming agent - the silk protein - meets the essential requirement of being water-soluble and biocompatible, to be used as the foaming template for macroporous CPSs. The benefit of this method over using porogens, detergents and/or salts in order to generate macroporosity is that the biocompatibility and injectability of the CPSs are not undermined by the use of the silk in order to achieve interconnectivity of the pores.

Despite the advantages of CPSs of being injectable and able to self-harden under physiological conditions, disintegration and release of fragments in contact with blood or other body fluids leading to embolism is a severe drawback in clinical practice. Previous studies have pointed out that one of the approaches to enhance cohesion of CPSs is by addition of a viscosity-enhancing agent, a polymeric additive for instance. Another plausible mechanism is through interaction of calcium ions with functional groups such as carboxylic groups in the additives, which gives rise to enhanced inter-crystal attraction, resulting in improvement on cement cohesion. It was surprisingly found thas the effect of silk enhancing the cohesion of CPSs are likely to lie at the basis of both criteria. Furthermore, the present invention is also based on the herein disclosed, surprising insights that by excluding detergents such as Tween 80, the cohesion time of silk-foamed CPSs (2 h) was even shorter than that of the dense controls (2 h 30 min, Table 2) at a constant L/P ratio, implying that the improved cohesion behavior was indeed originated from the presence of silk, rather than the compromised reactivity resulting from the low macroporosity and surface area compared to the Tween 80-foamed specimens.

In a preferred embodiment said method for producing a macroporous scaffold a powder form of the mineral phase is blended into the foamed liquid phase in step b).

The liquid foam is stabilized by incorporation of powder, which reduces the gravitational drainage and air diffusion between the bubbles. The porous structures are maintained through solidification of the cement. Furthermore, the inventors surprisingly found that silk-incorporated CPSs showed a dependence of compressive strength on macroporosity. As a matter of fact, a significant decrease in compressive strength was observed for Tween 80-foamed CPSs compared to the silk-foamed specimens in the present study, given that a two-fold higher macroporosity was achieved for the former.

Specimens foamed with silk (with a concentration of 3 mg/ml) showed lower macroporosity compared to Tween 80 but higher than the dense controls at the same L/P ratio. Despite that all of the samples presented intrinsic microporosity, more compact structures were shown by silk-incorporated CPSs compared to both the Tween 80-foamed specimens and the dense controls. While CPSs prepared with surfactant exhibited basically plate-like crystal morphology, a mixture of needle and plate-like structures were observed for silk incorporated cements, suggesting that the nucleation process was regulated by silk.

Apart from the foaming agent, Liquid-to-Powder (L/P) ratio may be another parameter of vital importance concerning the macroporosity of the CPSs. The porosity and microstructure of CPSs may be closely related to the L/P ratio and the particle size of the starting powder. Typical L/P ratios are in the range of 0.1 - 1.0, preferably 0.4 - 0.7, e.g. 0.5 or 0.65. As dissolution and crystal precipitation occur initially at the surface of the original particles, CPSs prepared at high L/P ratio may tend to show increased porosity, owing to enlarged distance between powder particles. With increasing L/P ratio, less powder is added, resulting in less shear forces required for mixing, thereby limiting the disruption of the foam structure, giving higher macroporosity in the end.

In a preferred embodiment, the mineral phase is comprising a retardant, such as sodium pyrophosphate (SPP). The retardant delays the setting of the composition to ensure workability thereof. A suitable concentration of the retardant in the mineral phase is 1 - 10 wt%, preferably 3-7 wt%, e.g. approximately 5 wt%.

In one preferred embodiment, the mineral phase is comprising an accelerator, such as Na₂HPO₄·2H₂O (NaP). The presence of the accelerator NaP in the mineral phase accelerated the reaction and surprisingly improved the porosity. A suitable concentration of the accelerator in the mineral phase is 0.5 - 5 wt%, preferably 2-3 wt%, e.g. approximately 2.5 wt%.

In the subsequent step (c), the macroporous paste is stabilized into a desired shape. This implies that cement cohesion of the paste occurs. The stabilized paste stays integrated and resistant to washout.

The pastes can e.g. be transferred into a silicon mold of desired shape and placed in a humid atmosphere at 37°C for 16 h to ensure cohesion.

Stabilization of the paste occurs at temperatures at or slightly above room temperature, and increasing temperature may be used to accelerate the speed of the reaction to induce faster formation of provided compositions.

In one embodiment, step c) is performed at 15-40°C. A preferred temperature range is room temperature, such as 15-25°C or 20-25°C. This facilitates the stabilizing step and allows for molding into various shapes. Another preferred temperature range is around body temperature, such as 35-40°C. This is useful for *in vivo* applications and allows for molding into various shapes.

In the subsequent step (d), the stabilized, shaped porous paste is mineralized into a macroporous calcium-deficient hydroxyapatite (CDHA)-based scaffold.

Mineralization of the stabilized, shaped paste can e.g. be achieved by immersing it in phosphate buffered saline (PBS) for 7 days to allow complete setting.

Mineralization of the stabilized, shaped paste occurs at temperatures at or slightly above room temperature, and increasing temperature may be used to accelerate the speed of the reaction to induce faster formation of provided compositions.

In one embodiment, step d) is performed at 15-40°C. A preferred temperature range is room temperature, such as 15-25°C or 20-25°C. This facilitates the mineralizing step and allows for fixation into various shapes. Another preferred temperature range is around body temperature, such as 35-40°C. This is useful for *in vivo* applications.

The method for producing a macroporous scaffold disclosed herein is advantageous in that it does not comprise any high-temperature sintering step, i.e. treatment at temperatures above 800 °C, involving the silk protein.

The method for producing a macroporous scaffold disclosed herein is also advantageous in that it does not involve addition of any non-biocompatible components such as detergents, solubilizing agents, and/or synthetic surfactants. Avoiding non-biocompatible components is highly desirable not to cause significant harm to living tissue when placed in contact with such tissue, e.g. *in vivo* and/or cause cell death, induce inflammation and/or other such adverse effects when administered *in vivo.*

In one aspect, said method for producing a macroporous scaffold as disclosed herein is an *in vitro* method.

Using *in vitro* methods allows for the possibilty to investigate the ability of spider silk protein to serve as a foaming agent to produce novel macroporous CPS scaffolds for bone regeneration and to further study the synergies between silk and CPSs.

Successful bone regenerative scaffolds are expected to have a high porosity with interconnected macropores which support cell migration and vascularization while the implants are progressively degraded and replaced by newly-formed tissue.

These biomolecules not only offer desirable foaming capacity, but also biological recognition *in vivo,* thereby promoting cell adhesion and bone ingrowth.

CPSs may have similar compositions to the mineral phase of naturals bones, and may thus be resorbable *in vivo.*

In another aspect, the macroporous paste is for use in a method for bone tissue engineering, wherein it is implanted into a human or an animal prior to step (d).

In a further aspect, the macroporous paste is for use in a method for bone tissue engineering, wherein it is implanted into a human or an animal prior to step (c).

In one aspect, the macroporous paste is for use in a method for bone tissue engineering, wherein it is injected into a human or an animal prior to step (c).

In a specific embodiment of these aspects, steps (c) and (d) are performed *in situ* in a human or an animal.

These features are highly advantageous, making the macroporous scaffold extremely appealing as bone void-fillers as they allow for minimal invasive procedure, and are adaptable to irregular cavities, avoiding gaps between scaffolds and the host tissue. Once injected, the macroporous paste may self-harden *in situ* through an isothermic setting reaction at physiological temperature. The self-setting reaction may occur in a dissolution/precipitation process: 1) dissolution of the powder upon mixing with the liquid phase, leading to supersaturation; 2) nucleation induced by supersaturation; 3) subsequent growth and precipitation of entangled crystals, resulting in macroscopic hardening of the cement.

According to a second aspect of the invention, an *in vitro* method for producing a macroporous paste is provided.

The inventors surprisingly found that silk holds the potential to serve as the foaming template for macroporous CPSs. The handling properties of CPSs were improved by incorporation of silk, showing positive effect on the cement behavior. The compositions of CPSs were also maintained in the presence of silk. The results of this invention shed light on a potential porous scaffold material for bone tissue engineering.

The present invention will now be further illustrated by the following non-limiting examples.

### EXAMPLES

### Materials and Methods

### Synthesis of calcium phosphate materials

Alpha-Tricalcium Phosphate (α-Ca₃(PO₄)₂, α-TCP) was synthesized by heating the mixture of calcium hydrogen phosphate (CaHPO₄, Sigma- Aldrich, USA) and calcium carbonate (CaCO3, Acros Organics, USA) with a 2:1 molar ratio at 1400 °C for 2-15 h followed by quenching in the air. The powder obtained was milled in a planetary ball mill (PM400, Retsch, Germany) for 15 min at 300 rpm. For α-TCP, 2 wt % of hydroxyapatite seed (HA, Merck, Germany) was added after milling to provide nucleation sites for hydroxyapatite crystals.

In addition, some preliminary studies were performed with and without the addition of 2.5 wt % Na₂HPO₄·2H₂O (NaP) to modify the pH and accelerate the reaction. This powder was used as the solid phase for preparation of calcium phosphate cements (CPC).

### Preparation of silk protein

A silk protein functionalized with a RGD-motif from fibronectin (FN-silk; ("FN_{cc}-4RepCT") having the amino acid sequence of SEQ ID NO: 3 was used.

### Preparation of Calcium Phosphate Scaffolds

Different parameters were applied to manufacture CPSs as following:
1) CDHA was prepared through hydrolysis of α-TCP.
2) two L/P ratio: 0.50 and 0.65 mL·g-1;
3) two types of foaming agents: Tween 80 (Sigma-Aldrich, USA) and silk. A consistent concentration of 1 wt % of Tween 80 was used as the control. For silk, three different concentrations were used to prepare macroporous CPSs: 1.5 mg/ml, 2 mg/ml, 3 mg/ml;
4) two consolidation methods: direct foaming for macroporous CPSs and manually mixing for dense specimens.
   - Direct foaming was achieved through mechanical stirring introduced by a domestic mixer to the liquid phase. The liquid phase was foamed for 5 s before the solid phase was added, followed by 25 s of mixing to obtain microporous scaffolds. Alternatively, the liquid phase was foamed mechanically for 5-7 seconds, and the powder was added in three portions, followed by 3-5 s of mixing/foaming.
   - Dense CPSs were prepared by manually mixing the powder and liquid phase (1 wt % Tween 80) in a mortar for 30 s.
5) After mixing, cement pastes were transferred immediately into silicon molds with two dimensions: Ø 12 × height 1 mm and Ø 6 × height 1 mm. Samples were placed in a humid atmosphere, e.g. for 16 h, at 37 °C to ensure cohesion, and subsequently immersed in 1× PBS solution (Sigma-Aldrich, USA) for 7-10 days to allow complete setting.

### Characterization of CPSs

The CPS scaffolds were characterized in terms of composition, cohesion time, porosity, morphology, specific surface area, and compressive strength.

Phase characterization was performed using a diffractometer (D5000 Th-Th, Siemens, Germany) equipped with a Cu Kα anode. Data were collected over 2θ range from 10° to 80° at steps of 0.02° per second.

Phase identification was achieved by comparing the patterns obtained with the standard of α-TCP (JCPDS 04-010-4348), β-TCP (JCPDS 04-008-8714), HA (JCPDS 01-074-0565) brushite (JCPDS 04-013-3344) and monetite (JCPDS 04-009-3755).

Semi-quasi quantitative analysis was done by DIFFRAC.EVA (Bruker, USA). Chemical composition was characterized by attenuated total reflection-fourier-transform infrared spectroscopy (ATR-FTIR, Tensor 27, Bruker, USA). Transmittance spectra were obtained in the range of 400-4000 cm⁻¹ with 1024 scans and a resolution of 2 cm⁻¹ for silk-incorporated CPSs. For silk-free samples, spectra were obtained in the same range using 64 scans and resolution of 4 cm⁻¹.

Cohesion time of CPSs at 37 °C was determined by addition of 0.5 mL PBS solutions to cement paste filled into a well-plate using a spatula at a set time interval of 30 min.

Macroporosity of the scaffolds was assessed by microcomputed tomopography (micro-CT, Skyscan, Bruker, USA). Images were acquired with pixel size of 10 µm at a voltage of 100 kV and a current of 100 µA with an Al-Cu filter. Reconstruction was performed using NRecon (Bruker, USA) and calculations were done by CTAn software (Bruker, USA) to acquire macroporosity.

The morphology and microstructure of CPSs were studied by scanning electron microscope (SEM, LEO 1550 Gemini, Carl Zeiss Meditec AG, Germany). Samples were sputtered with a thin gold coating for 40 s prior to operation. For each sample, surface and fractured cross-section were analyzed. The specific surface area was analyzed using Brunauer-Emmett-Teller (BET) method (ASAP 2020, Micrometrics Instrument Corp., USA).

The compressive strength was measured according to the ISO 5833 standard. The mechanical testing machine (Shimadzu AGS-X, Japan) was operated with a 5 kN load cell at a testing speed of 1 mm/min until failure. Samples were molded into cylindrical silicon molds with the dimension of Ø 6 × height 12 mm and stored in PBS at 37 °C for 10 days prior to measurement.

### Handling Properties of CPSs

The cohesion time of CPSs prepared with a constant L/P ratio of 0.65 ml/g are summarized in Table 1. The silk-foamed samples were prepared with a concentration of 3 mg/ml. Effect of consolidation method on cohesion of CPSs were studied between the dense and the Tween 80-foamed specimens. The dense controls were cohesive after 2 h 30 min of setting, which was considerably shorter compared to 3 h 30 min for the foamed ones due to the lower surface area and reduced reactivity. Regarding the influence of the foaming agent, silk was found to be able to promote cement cohesion, with silk-foamed CPSs showing cohesiveness after only 2 h, which was faster than the specimens prepared with Tween 80 solution regardless of the consolidation process.

**Table 1. Cohesion time of CPSs. All of the samples were prepared at L/P=0.65 ml/g. For CPSs prepared with Tween 80 solution as the liquid phase, the dense controls had shorter cohesion time than the foamed specimens. Silk was found to promote cement cohesion, exhibiting fastest cohesion among all samples.**

| Sample | Foaming agent | Consolidation method | Cohesion time |
|---|---|---|---|
| Surfactant-foamed CPS | Tween 80 | Direct foaming | 3 h 30 min |
| Silk-foamed CPS | Silk (3 mg/ml) | Direct foaming | 2 h |
| Dense CPS | Tween 80 | Manually mixing | 2 h 30 min |

### Chemical Characterization

The effect of foaming agent, L/P ratio, and consolidation method on the compositions of the CPSs were investigated by XRD and ATR-FTIR.

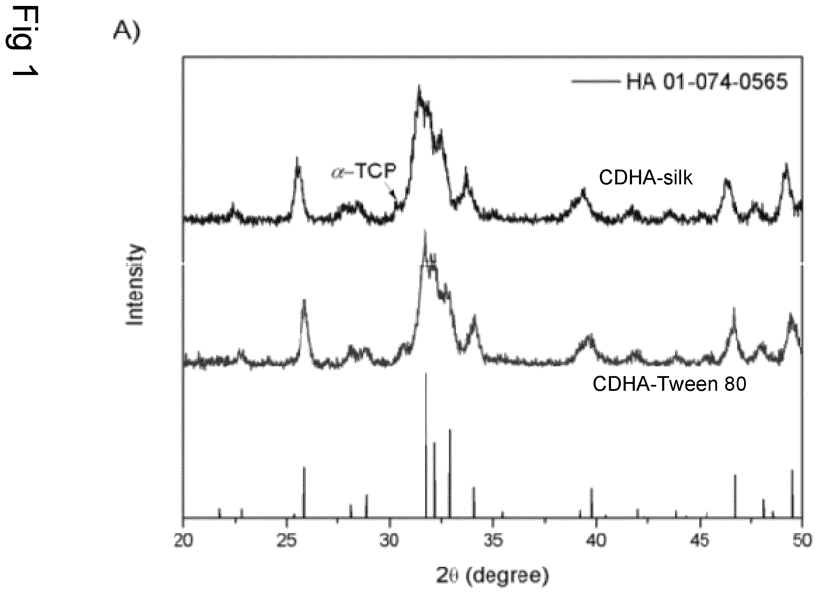

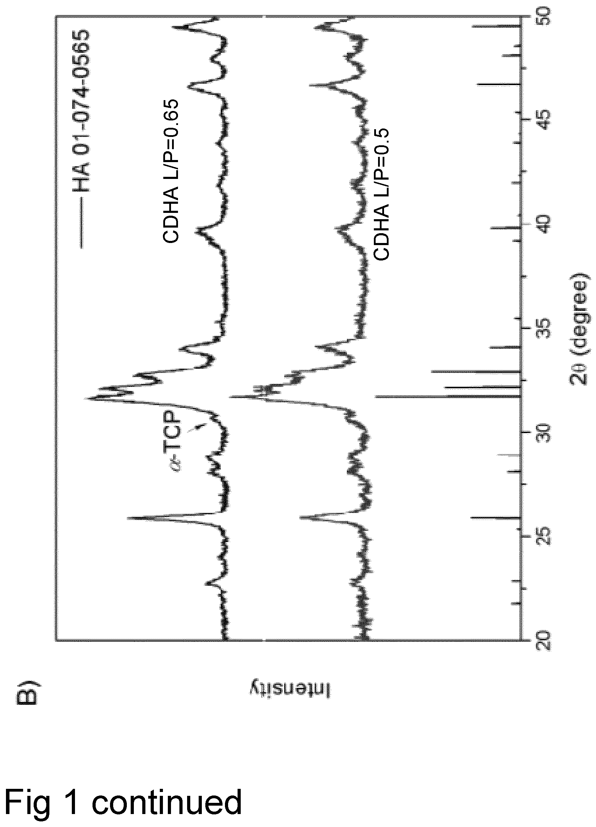

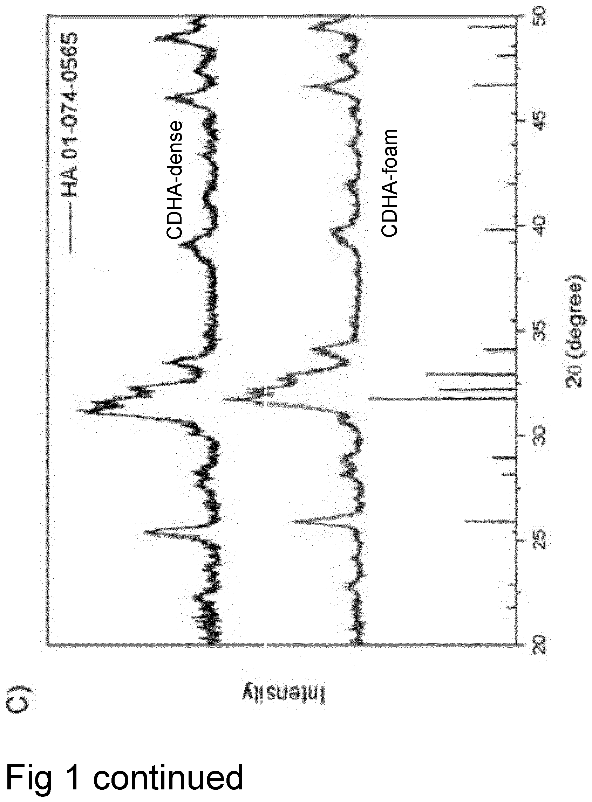

Phase analysis by XRD revealed that the final compositions were predominantly CDHA, in spite of the different preparation protocols (shown in Figure 1). The CPSs prepared were poorly crystallized, showing broadened peaks. Incorporation of silk (with a concentration of 3 mg/ml) to the CPSs yielded slightly broader peaks than the surfactant given the same L/P ratio of 0.5 ml/g, indicating that finer crystals were formed in the presence of silk, which was further confirmed by microscopy (Figure 1A; upper line: CDHA-silk; lower line CDHA-Tween 80). For CPSs foamed with Tween 80, specimens with L/P ratio of 0.65 showed a sharper peak at 25.88°, yet no significant difference could be observed in the overall crystallinity (Figure 1B; upper line: CDHA L/P=0.65; lower line CDHA L/P=0.5). Similar patterns were obtained for CPSs prepared by different consolidation method with Tween 80 solution at a fixed L/P ratio of 0.5, which suggested that the consolidation process did not have any profound effect on the phase composition of the cements (Figure 1C; upper line: CDHA-dense; lower line CDHA-foam).

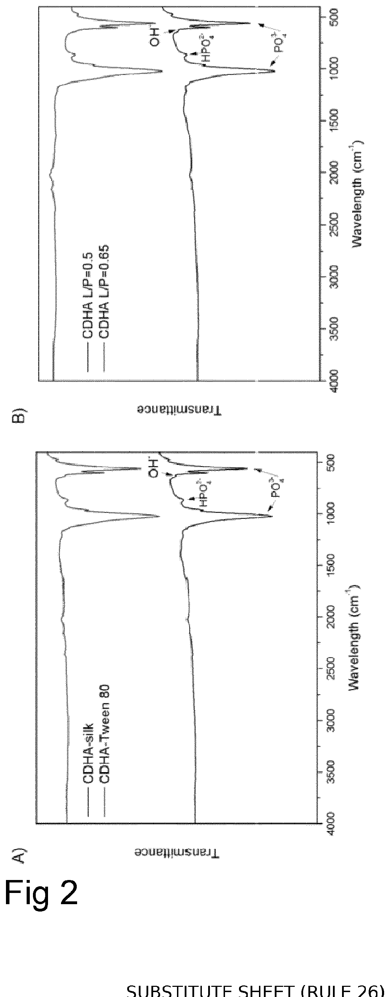

Infrared spectroscopy performed by ATR-FTIR confirmed that the chemical composition of CPSs was maintained consistent regardless of the foaming agent or L/P ratio used (shown in Figure 2). Figure 2 shows ATR-FTIR spectra of CPSs prepared with (A) silk (lower line) and Tween 80 (upper line) as the foaming agent at L/P=0.5 ml/g; (B) Tween 80 solution at L/P ratio of 0.5 (lower line) and 0.65 g/ml/g (upper line). The bands detected at 470, 560-600, 964 and 1020~1120 cm⁻¹ corresponded to v2, v3, v1 and v4 mode of PO₄³⁻ respectively. The band at around 630 cm⁻¹ was ascribed to OH⁻ in CDHA. The minor band at around 850~880 cm⁻¹ was assigned to HPO₄²⁻.

### Results

The consolidation method did not have sound effect on the composition. Typical amide bands were not able to be identified for CPSs prepared with silk, due to the low amount of protein (3 mg/ml). Besides, significant decrease in intensity of the three amide peaks could be provoked by interaction of carbonyl groups (>C=O) in amide bonds with calcium ions.

### Morphological Characterization

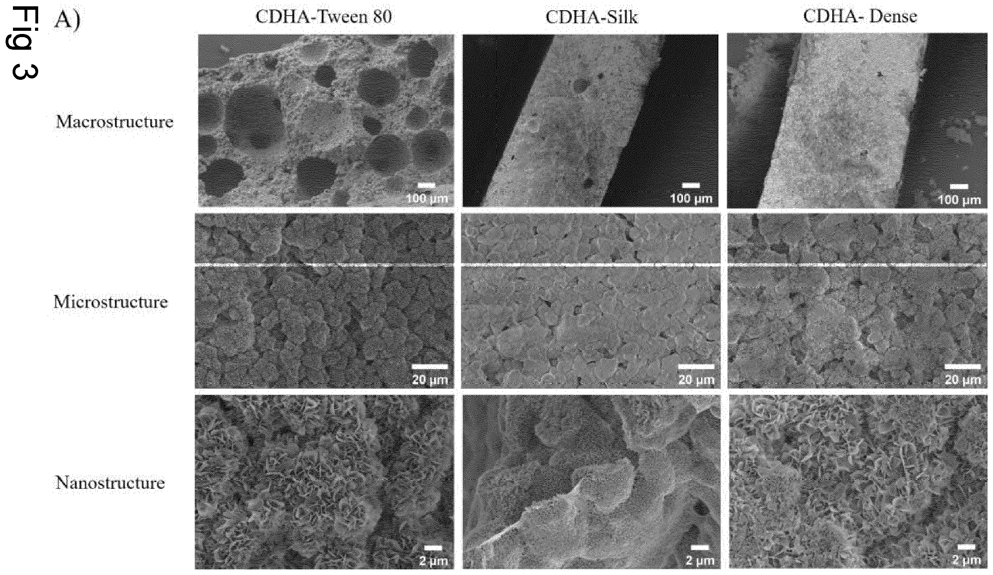

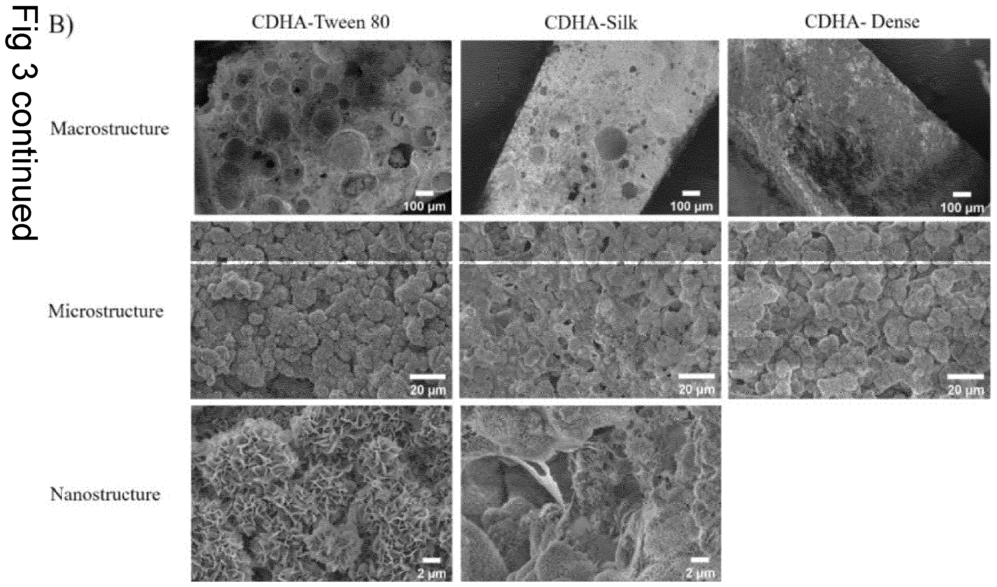

Morphological analysis was performed by SEM (Figure 3) to investigate the effect of foaming agent, L/P ratio and consolidation method on the structural properties of the CPSs.

Fig 3 shows microscopic images of the CPSs prepared at (A) L/P=0.5 ml/g; (B) L/P=0.65 ml/g. Specimens foamed with silk (with a concentration of 3 mg/ml) showed lower macroporosity compared to Tween 80 but higher than the dense controls at the same L/P ratio. Despite that all of the samples presented intrinsic microporosity, more compact structures were shown by silk-incorporated CPSs compared to both the Tween 80-foamed specimens and the dense controls. While CPSs prepared with surfactant exhibited basically plate-like crystal morphology, a mixture of needle and plate-like structures were observed for silk incorporated cements, suggesting that the nucleation process was regulated by silk.

For Tween 80-foamed CPSs, specimens with L/P ratio of 0.5 ml/g were less porous than 0.65 ml/g on macro-scale, which was later confirmed by micro-CT (shown in the Appendix). CPSs prepared at L/P=0.5 ml/g presented mainly large pores, while those prepared at L/P=0.65 ml/g had a mixture of pores with different sizes, showing a less compact architecture. Enlarged inter-aggregate spaces were found in the microstructures of the samples prepared at a higher L/P ratio. The nanostructure was not affected by the different L/P ratios. Few macropores could be observed for the dense controls, yet the microporosity remained, due to that CPSs are porous materials and the inherent porosity was independent on the consolidation process.

Macroporosity of the CPSs was assessed by micro-CT to investigate the performance of silk as macroporous template for bone cement scaffolds. All of the samples were prepared at L/P=0.65 ml/g, aiming for potentially higher porosity. As shown in Figure 4, the macroporosity of CPSs foamed with silk concentrations of 1.5, 2 and 3 mg/ml were 8.19±1.77%, 10.69±2.23% and 9.93±1.19% respectively. Optimal porosity was achieved with silk concentration of 2 mg/ml, yet the values were similar among different concentrations. In addition, the highest macroporosity obtained for silk-foamed CPSs was still not comparable to the Tween 80-foamed specimens, which had a macroporosity of 22.13±0.69%. The dense controls had the lowest porosity (0.53±0.26%) among all CPSs, which was in agreement with the microscopic analysis.

The specific surface area (SSA) of the CPSs prepared at L/P ratio 0.65 ml/g was determined by BET as summarized in Table 2. The silk-foamed specimens were foamed with a concentration of 2 mg/ml based on the macroporosity analysis. Similar SSA values were acquired among the three samples (~25 m²/g), showing that neither the foaming agent nor the consolidation method had any fundamental effect on the overall nanostructure of the CPSs.

**Table 2. BET specific surface area of the CPSs prepared at L/P=0.65. The silk concentration used for the silk-foamed specimens was 2 mg/ml. Values acquired for CPSs foamed with Tween 80 and silk, as well as the dense controls, were very similar, and relatively low in comparison with the literature**

| Sample | Surfactant-foamed CPS | Silk-foamed CPS | Dense control |
|---|---|---|---|
| SSA (m²/g) | 24.62 | 24.57 | 26.40 |

### Results

At the same L/P ratio, CPSs foamed with Tween-80 showed the highest macroporosity, while those foamed with silk presented fewer pores, showing slightly higher macroporosity than the dense controls. A more compact microstructure was observed in the presence of silk. In contrast, Tween 80-foamed CPSs and the dense controls displayed predominantly plate-like crystals, while silk-foamed CPSs had a mixture of needles and plates. For CPSs foamed with Tween 80, higher macroporosity and a wider pore size distribution was displayed at a higher L/P ratio. Increased inter-aggregate distance was found in the microstructure and L/P=0.65 ml/g. The nanostructure was not markedly affected. The dense controls showed compact structure in the macroscopic range, yet the textural properties were similar to the Tween 80-foamed specimens on micro/nanometric scale.

The porosity of the dense control was 0.53±0.26%, which was the lowest among all samples.

### Mechanical Properties

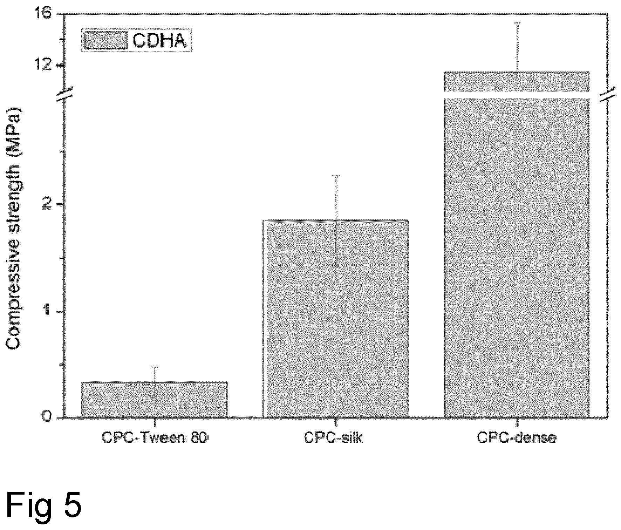

The compressive strength (CS) was measured for CPSs with L/P ratio of 0.65 ml/g, as shown in Figure 5. The Tween 80-foamed CPSs gave the lowest compressive strength of 0.33±0.17 MPa, as a result of the highest macroporosity. The specimens foamed with 2 mg/ml silk yielded stronger materials compared to Tween 80, with a CS value of 1.85±0.42 MPa. As expected, the dense control showed the highest CS of 11.53±3.87 MPa, given its low porosity and relatively compact structure.

### Results

The CS values were 0.33±0.17, 1.85±0.42, and 11.53±3.87 MPa respectively, which were in alignment with their macroporosity.

### Optimisation of the concentration of additives in the calcium phosphate foams (CPFs)

In a preliminary study, samples of silk-CPF with a silk concentration of 2 mg/ml in the liquid phase showed to provide the best stability of the macroporous template. Therefore, these samples were used as a reference when optimising the concentration of Tween in the Tween-CPF samples, with the aim to obtain two CPF materials with a similar distribution of macropores. In addition, the effect in the porosity of the presence of NaP in the solid phase was evaluated for both silk-CPF and tween-CPF. For this optimisation process, the porosity of the materials was analysed in terms of total porosity (uCT) and visualized by SEM.

The composition selected for the CPF samples was 2 mg/ml silk and 0.1 w/v% Tween, both of them containing NaP in the solid phase.

### Results

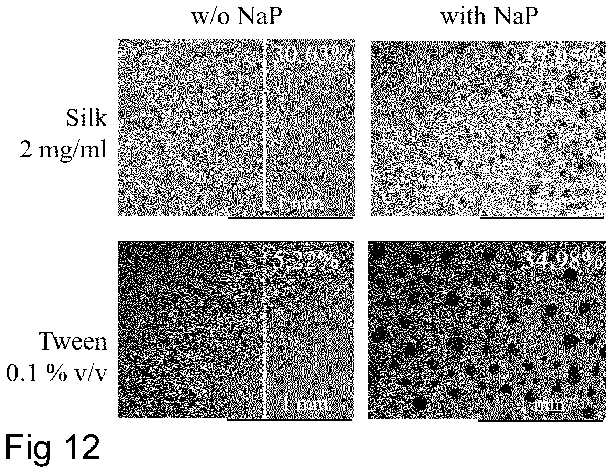

The presence of NaP in the solid phase improved the surface porosity, as shown in Figure 12.

### Cell data on Silk-CDHA scaffolds and CDHA-controls

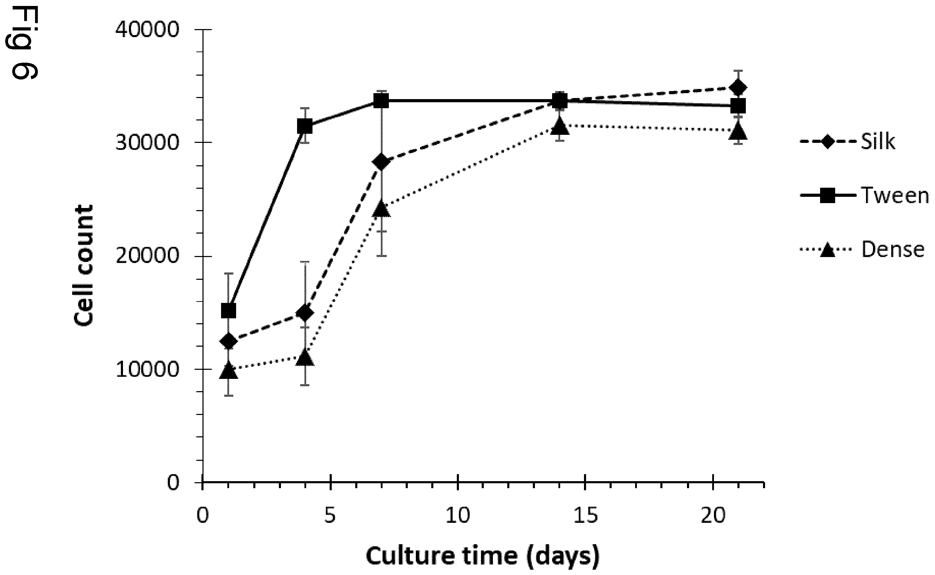

Mice mesenchymal (mMSC) cells cultured on CDHA were assayed for total lactate dehydrogenase activity to monitor cell growth (mean and standard deviation of 3 independent experiments). The results are shown in Figure 6.

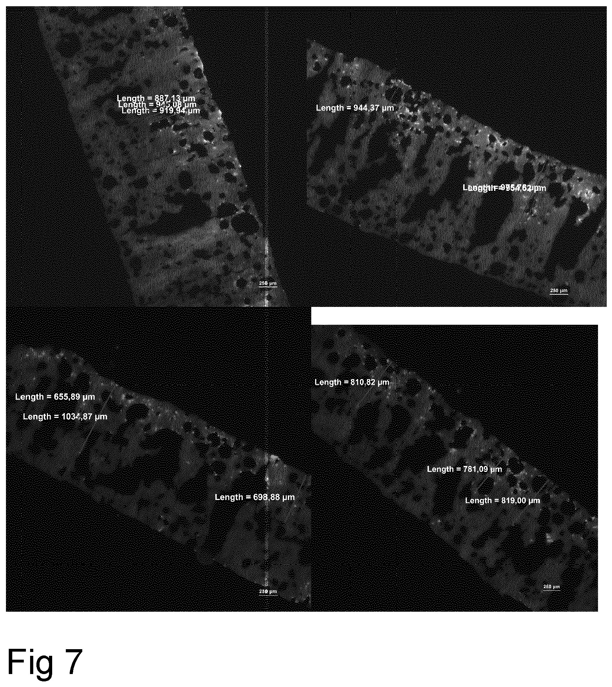

Cross sections of silk CDHA scaffolds were prepared after 3 weeks of culture with mMSCs. Figure 7 shows examples of the cross sections obtained. Lower panel shows cell nuclei; upper panel shows cytoskeleton.

### Results

It was shown that cells survive and grow well when cultured on both the Silk CDHA and the control CDHAs (tween and dense).

The cells migrated from the top of the scaffolds and approximately half way through.

### In vitro tests

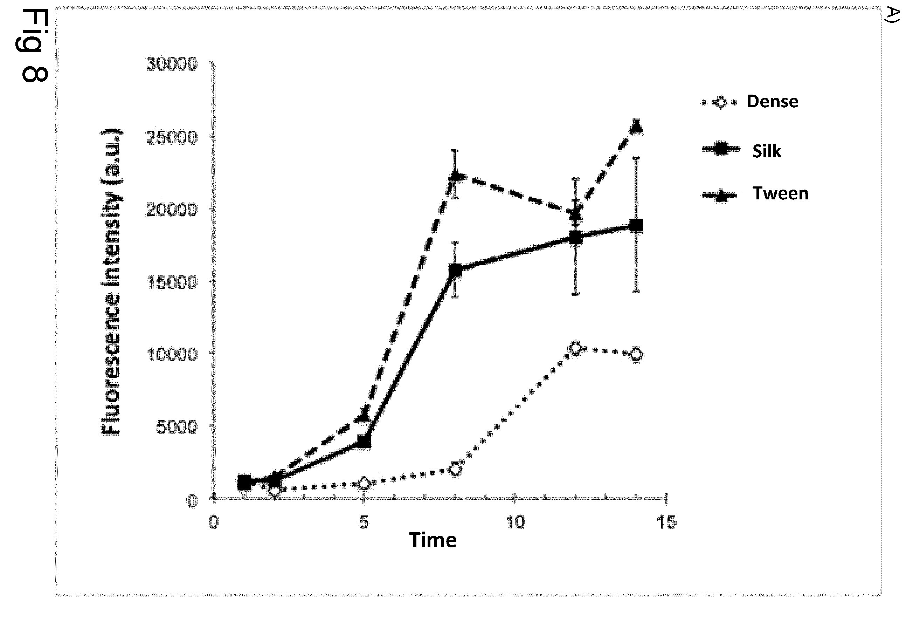

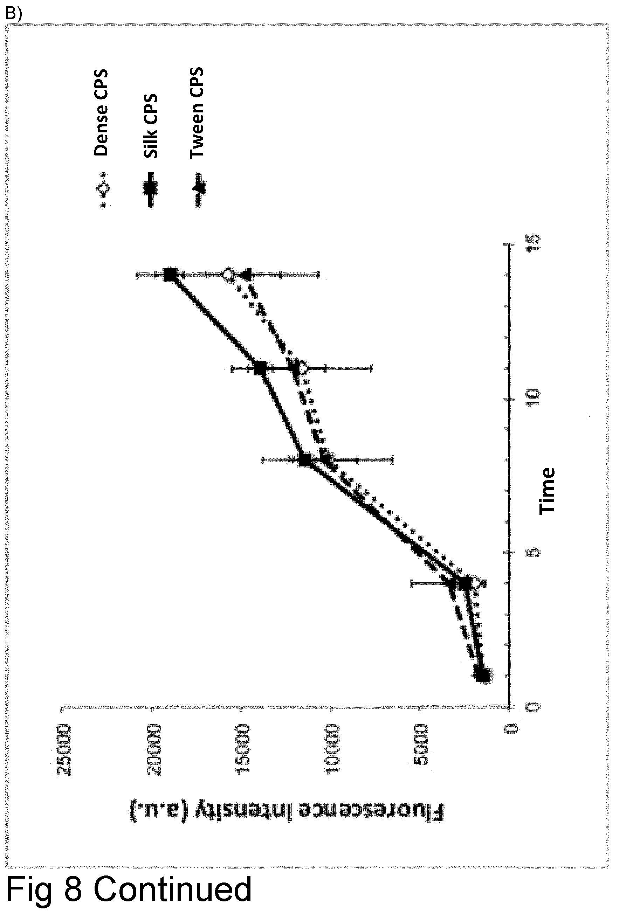

To investigate cell viability, CDHA CPSs were prepared at (A) L/P=0.5 ml/g; (B) L/P=0.65 ml/g seeded with 10×103 mice mesenchymal cells (mMSCs) per cm². The concentration of silk used for silk-foamed CPSs was 2 mg/ml. The results are shown in Figure 8.

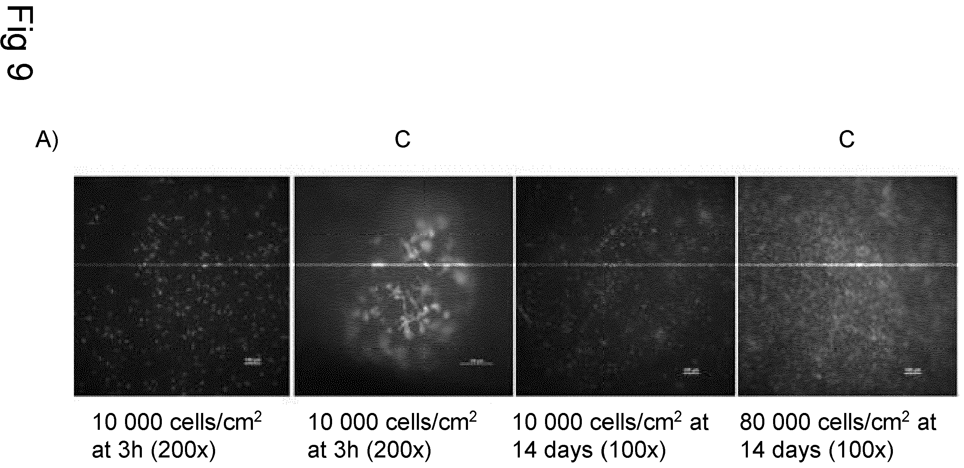

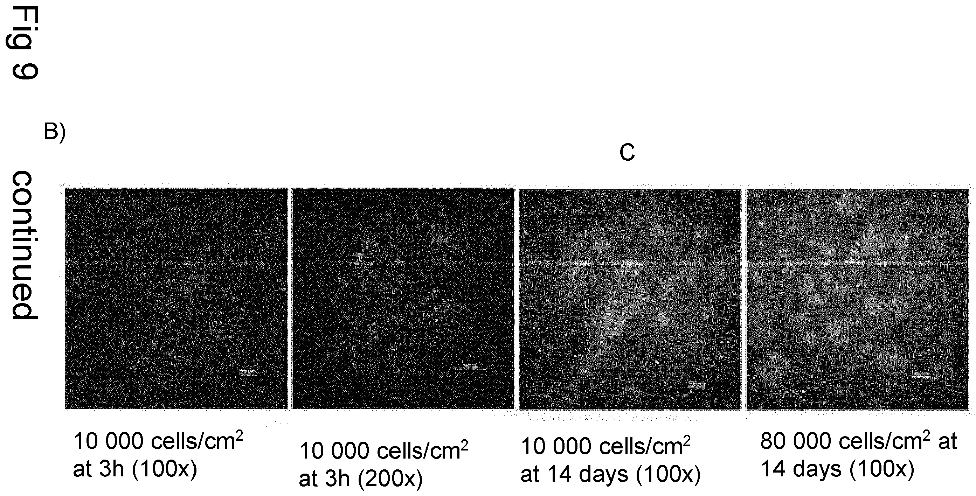

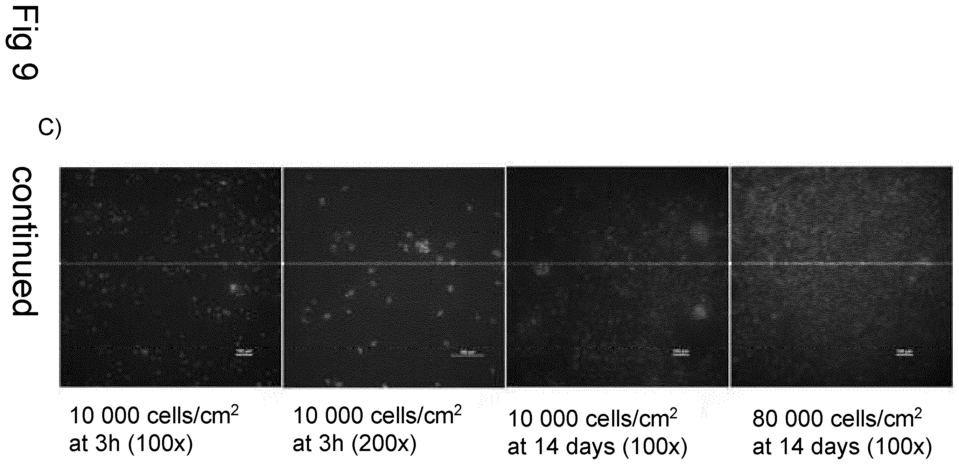

Fluorescence micrographs of (A) Tween 80-foamed CPSs, (B) silk-foamed CPSs, and (C) the dense controls with a L/P ratio of 0.5 ml/g at 3 h and 14 days were prepared. The micrographs are shown in Figure 9.

### Results

Figure 8 shows that at L/P=0.5 ml/g, cell viability on silk-foamed CPSs was lower than that on Tween-foamed CPSs, but results were comparable. At L/P=0.65 ml/g, cell viability on silk-foamed CPSs was higher than that on scaffolds foamed with Tween 80. It should be kept in mind that at both L/P ratios, the macroporosity of silk-foamed scaffolds was lower than the Tween 80-foamed specimens, indicating that silk had a positive effect on cell viability which compensated for the low macroporosity.

Early and late cell attachment, respectively, is shown in Figure 9. The cytoskeleton is marked with a C and while the rest depicts the cell. An extensive network of actin filaments was observed on silk-foamed CPSs at 3 h, while a more conservative cell morphology was found on Tween 80-foamed scaffolds and the dense control. After 14 days, similar cell morphologies were presented on both types of foamed CPSs, with silk and Tween 80, which were much more extensive than on the dense controls.

### Characterization of CDHA

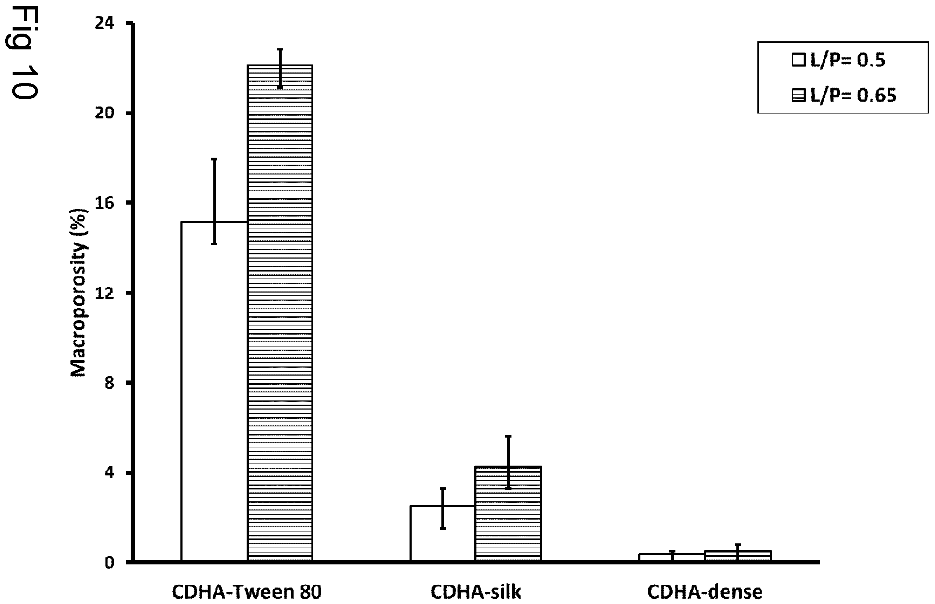

Macroporosity of CPSs were prepared with different L/P ratios. The silk-foamed CPSs were prepared with a concentration of 1 mg/ml silk, see Figure 10.

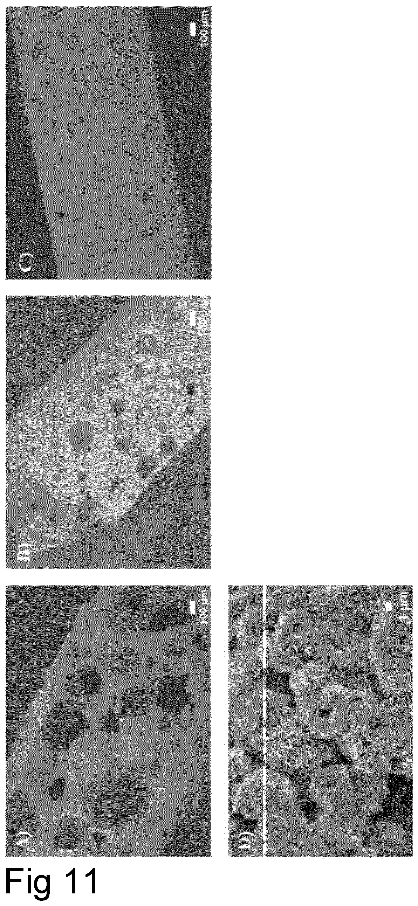

SEM was performed and images of the macrostructures are shown in Figure 11. Fig 11(A) shows CPSs foamed with Tween 80, Fig 11(B) shows CPSs foamed with 2 mg/ml silk, and Fig 11(C) shows dense CPSs as the control, corresponding to macroporosities of 22.13±0.69%, 10.69±2.23% and 0.53±0.26%, respectively. Fig 11(D) shows nanostructures of silk-foamed CPSs (with 1 mg/ml silk), showing a majority of plate-like crystals.

### Results

Figure 10 shows that the macroporosity was found to increase with increasing L/P ratio for the foamed samples (with Tween 80 and silk).

## Claims

1. An *in vitro* method for producing a macroporous scaffold, comprising the steps:
(a) subject a liquid phase which is an aqueous solution of a non-denatured silk protein to mechanical foaming, wherein the non-denatured silk protein is the foaming agent and wherein the non-denatured silk protein is capable of assembling into a water-insoluble macrostructure at a water/air interface;
(b) blend a mineral phase comprising α-tricalcium phosphate (α-TCP) with hydroxyapatite (HA) into the foamed liquid phase during continued mechanical foaming, thereby forming a macroporous paste;
(c) stabilize the macroporous paste into a desired shape;
(d) mineralize the stabilized, shaped porous paste into a macroporous calcium-deficient hydroxyapatite (CDHA)-based scaffold;
wherein the method does not comprise any high-temperature sintering step; and wherein the method does not involve addition of detergents.

2. An *in vitro* method for producing a macroporous paste, comprising the steps:
(a) subject a liquid phase which is an aqueous solution of a non-denatured silk protein to mechanical foaming, wherein the non-denatured silk protein is the foaming agent and wherein the non-denatured silk protein is capable of assembling into a water-insoluble macrostructure at a water/air interface; and
(b) blend a mineral phase comprising α-tricalcium phosphate (α-TCP) with hydroxyapatite (HA) into the foamed liquid phase during continued mechanical foaming, thereby forming a macroporous paste;
wherein the method does not comprise any high-temperature sintering step; and wherein the method does not involve addition of detergents.

3. A method according to claim 1, wherein step c) and/or step d) is performed at 15-40°C.

4. A method according to any previous claim, wherein a powder form of the mineral phase is blended into the foamed liquid phase in step b).

5. A method according to any previous claim, wherein the mineral phase is comprising a retardant.

6. A method according to claim 5, wherein the retardant is sodium pyrophosphate.

7. A method according to any previous claim, wherein the mineral phase is comprising an accelerator.

8. A method according to claim 7, wherein the accelerator is Na₂HPO₄·2H₂O (NaP).

9. A method according to any previous claim, wherein the method does not involve addition of any non-biocompatible components.

10. A method according to any previous claim, wherein the silk protein is a recombinant silk protein.

11. A method according to any previous claim, wherein the silk protein is a spider silk protein.

12. A method according to any previous claim, wherein the silk protein contains a cell-binding motif (CBM) as a functional moiety.

13. A macroporous paste comprising a foamed blend of (i) a pre-foamed, non-denatured silk protein foaming agent and (ii) a mineral phase comprising α-tricalcium phosphate (α-TCP) with hydroxyapatite (HA); wherein the macroporous paste is non-sintered and wherein the macroporous paste does not comprise any detergents.

14. A macroporous paste according to claim 13, as further defined in any one claims 4-8 or 10-12.

15. A macroporous paste obtainable by the method of claim 2, as further defined in any one of claims 3-12.

16. A macroporous paste according to any one of claims 13 to 15 for use in a method for bone tissue engineering, the method comprising the steps:
(c1) implant or inject the macroporous paste into a human or an animal;
(c2) stabilize the macroporous paste into a desired shape *in situ;*
(d) mineralize the stabilized, shaped porous paste into a macroporous calcium-deficient hydroxyapatite (CDHA)-based scaffold *in situ;*
wherein the method does not comprise any high-temperature sintering step; and wherein the method does not involve addition of detergents.

17. A macroporous paste according to any one of claims 13 to 15 for use in a method for bone tissue engineering, the method comprising the steps:
(c2) stabilize the macroporous paste into a desired shape *in vitro;*
(c1) implant the stabilized, shaped macroporous paste into a human or an animal;
(d) mineralize the stabilized, shaped porous paste into a macroporous calcium-deficient hydroxyapatite (CDHA)-based scaffold *in situ;*
wherein the method does not comprise any high-temperature sintering step; and wherein the method does not involve addition of detergents.

18. Use of a non-denatured silk protein as a foaming agent in a macroporous paste suitable for obtaining macroporous calcium-deficient hydroxyapatite (CDHA)-based scaffolds.

19. Use according to claim 18, wherein the silk protein is a spider silk protein.

20. Use according to any one of claims 18-19, wherein the silk protein contains a cell-binding motif (CBM) as a functional moiety.

## Patentansprüche

1. In-vitro-Verfahren zur Herstellung eines makroporösen Gerüsts, umfassend die Schritte:
(a) Unterwerfen einer flüssigen Phase, die eine wässrige Lösung eines nicht denaturierten Seidenproteins ist, an mechanisches Schäumen, wobei das nicht denaturierte Seidenprotein der Schaumbildner ist und wobei das nicht denaturierte Seidenprotein fähig ist, sich zu einer wasserunlöslichen Makrostruktur an einer Wasser/Luft-Grenzfläche zusammenzusetzen;
(b) Mischen einer Mineralphase, die α-Tricalciumphosphat (α-TCP) mit Hydroxylapatit (HA) umfasst, unter fortgesetztem mechanischem Schäumen in die geschäumte flüssige Phase, um eine makroporöse Paste zu bilden;
(c) Stabilisieren der makroporösen Paste zu einer gewünschten Form;
(d) Mineralisieren der stabilisierten, geformten porösen Paste zu einem makroporösen Gerüst auf der Basis von calciumdefizientem Hydroxyapatit (CDHA);
wobei das Verfahren keinen Hochtemperatursinterschritt umfasst;
und wobei das Verfahren keine Zugabe von Detergenzien umfasst.

2. In-vitro-Verfahren zur Herstellung einer makroporösen Paste, umfassend die Schritte:
(a) Unterwerfen einer flüssigen Phase, die eine wässrige Lösung eines nicht denaturierten Seidenproteins ist, an mechanisches Schäumen, wobei das nicht denaturierte Seidenprotein der Schaumbildner ist und wobei das nicht denaturierte Seidenprotein fähig ist, sich zu einer wasserunlöslichen Makrostruktur an einer Wasser/Luft-Grenzfläche zusammenzusetzen; und
(b) Mischen einer Mineralphase, die α-Tricalciumphosphat (α-TCP) mit Hydroxylapatit (HA) umfasst, unter fortgesetztem mechanischem Schäumen in die geschäumte flüssige Phase, um eine makroporöse Paste zu bilden;
wobei das Verfahren keinen Hochtemperatursinterschritt umfasst;
und wobei das Verfahren keine Zugabe von Detergenzien umfasst.

3. Verfahren nach Anspruch 1, wobei Schritt c) und/oder Schritt d) bei 15-40 °C durchgeführt wird.

4. Verfahren nach einem der vorstehenden Ansprüche, wobei bei Schritt b) eine Pulverform der Mineralphase in die geschäumte flüssige Phase gemischt wird.

5. Verfahren nach einem der vorstehenden Ansprüche, wobei die Mineralphase ein Verzögerungsmittel umfasst.

6. Verfahren nach Anspruch 5, wobei das Verzögerungsmittel Natriumpyrophosphat ist.

7. Verfahren nach einem der vorstehenden Ansprüche, wobei die Mineralphase einen Beschleuniger umfasst.

8. Verfahren nach Anspruch 7, wobei der Beschleuniger Na₂HPO₄·2H₂O (NaP) ist.

9. Verfahren nach einem der vorstehenden Ansprüche, wobei das Verfahren keine Zugabe von nicht biokompatiblen Komponenten umfasst.

10. Verfahren nach einem der vorstehenden Ansprüche, wobei das Seidenprotein ein rekombinantes Seidenprotein ist.

11. Verfahren nach einem der vorstehenden Ansprüche, wobei das Seidenprotein ein Spinnen-Seidenprotein ist.

12. Verfahren nach einem der vorstehenden Ansprüche, wobei das Seidenprotein ein zellbindendes Motiv (CBM) als funktionelle Einheit enthält.

13. Makroporöse Paste, umfassend ein geschäumtes Gemisch von (i) einem vorgeschäumten, nicht denaturierten Seidenprotein-Schaumbildner und (ii) einer Mineralphase umfassend α-Tricalciumphosphat (α-TCP) mit Hydroxyapatit (HA); wobei die makroporöse Paste nicht gesintert ist und wobei die makroporöse Paste keine Detergenzien umfasst.

14. Makroporöse Paste nach Anspruch 13, wie weiter definiert in einem der Ansprüche 4-8 oder 10-12.

15. Makroporöse Paste, erhältlich durch das Verfahren nach Anspruch 2, wie weiter definiert in einem der Ansprüche 3-12.

16. Makroporöse Paste nach einem der Ansprüche 13 bis 15 zur Verwendung bei einem Verfahren zum Knochengewebe-Engineering, wobei das Verfahren die Schritte umfasst:
(c1) Implantieren oder Injizieren der makroporösen Paste in einen Menschen oder ein Tier;
(c2) Stabilisieren der makroporösen Paste in situ zu einer gewünschten Form;
(d) Mineralisieren der stabilisierten, geformten porösen Paste in situ zu einem Gerüst auf der Basis von makroporösem calciumdefizienten Hydroxyapatit (CDHA);
wobei das Verfahren keinen Hochtemperatursinterschritt umfasst;
und wobei das Verfahren keine Zugabe von Detergenzien umfasst.

17. Makroporöse Paste nach einem der Ansprüche 13 bis 15 zur Verwendung bei einem Verfahren zum Knochengewebe-Engineering, wobei das Verfahren die Schritte umfasst:
(c2) Stabilisieren der makroporösen Paste in vitro zu einer gewünschten Form;
(c1) Implantieren der stabilisierten, geformten makroporösen Paste in einen Menschen oder ein Tier;
(d) Mineralisieren der stabilisierten, geformten porösen Paste in situ zu einem Gerüst auf der Basis von makroporösem calciumdefizienten Hydroxyapatit (CDHA);
wobei das Verfahren keinen Hochtemperatursinterschritt umfasst;
und wobei das Verfahren keine Zugabe von Detergenzien umfasst.

18. Verwendung eines nicht denaturierten Seidenproteins als Schaumbildner in einer makroporösen Paste, die zum Erhalten von makroporösen Gerüsten auf der Basis von calciumdefizientem Hydroxylapatit (CDHA) geeignet ist.

19. Verwendung nach Anspruch 18, wobei das Seidenprotein ein Spinnen-Seidenprotein ist.

20. Verwendung nach einem der Ansprüche 18-19, wobei das Seidenprotein ein zellbindendes Motiv (CBM) als funktionelle Einheit enthält.

## Revendications

1. Procédé *in vitro* de production d'un échafaudage macroporeux, comprenant les étapes :
(a) soumettre une phase liquide qui est une solution aqueuse d'une protéine de soie non dénaturée à un moussage mécanique, la protéine de soie non dénaturée étant l'agent moussant et la protéine de soie non dénaturée étant capable de s'assembler en une macrostructure insoluble dans l'eau au niveau d'une interface eau/air ;
(b) mélanger une phase minérale comprenant du phosphate α-tricalcique (α-TCP) avec de l'hydroxyapatite (HA) dans la phase liquide moussée pendant la poursuite du moussage mécanique, formant ainsi une pâte macroporeuse;
(c) stabiliser la pâte macroporeuse dans une forme souhaitée ;
(d) minéraliser la pâte poreuse mise en forme stabilisée en un échafaudage macroporeux à base d'hydroxyapatite carencée en calcium (CDHA);
dans lequel le procédé ne comporte aucune étape de frittage à haute température ;
et dans lequel le procédé n'implique pas un ajout de détergents.

2. Procédé de production *in vitro* d'une pâte macroporeuse comprenant les étapes :
(a) soumettre une phase liquide qui est une solution aqueuse d'une protéine de soie non dénaturée à un moussage mécanique, la protéine de soie non dénaturée étant l'agent moussant et la protéine de soie non dénaturée étant capable de s'assembler en une macrostructure insoluble dans l'eau au niveau d'une interface eau/air ; et
(b) mélanger une phase minérale comprenant du phosphate α-tricalcique (α-TCP) avec de l'hydroxyapatite (HA) dans la phase liquide moussée pendant la poursuite du moussage mécanique, formant ainsi une pâte macroporeuse;
dans lequel le procédé ne comporte aucune étape de frittage à haute température ;
et dans lequel le procédé n'implique pas un ajout de détergents.

3. Procédé selon la revendication 1, dans lequel l'étape c) et/ou l'étape d) est effectuée à 15-40 °C.

4. Procédé selon l'une quelconque des revendications précédentes, dans lequel une forme pulvérulente de la phase minérale est mélangée dans la phase liquide moussée dans l'étape b).

5. Procédé selon l'une quelconque des revendications précédentes, dans lequel la phase minérale comprend un retardateur.

6. Procédé selon la revendication 5, dans lequel le retardateur est le pyrophosphate de sodium.

7. Procédé selon l'une quelconque des revendications précédentes, dans lequel la phase minérale comprend un accélérateur.

8. Procédé selon la revendication 7, dans lequel l'accélérateur est Na₂HPO₄·2H₂O (NaP).

9. Procédé selon l'une quelconque des revendications précédentes, dans lequel le procédé n'implique pas l'ajout de composants non biocompatibles.

10. Procédé selon l'une quelconque des revendications précédentes, dans lequel la protéine de soie est une protéine de soie recombinante.

11. Procédé selon l'une quelconque des revendications précédentes, dans lequel la protéine de soie est une protéine de soie d'araignée.

12. Procédé selon l'une quelconque des revendications précédentes, dans lequel la protéine de soie contient un motif de liaison cellulaire (CBM) en tant que fragment fonctionnel.

13. Pâte macroporeuse comprenant un mélange moussé de (i) un agent moussant de protéine de soie non dénaturée prémoussé et (ii) une phase minérale comprenant du phosphate α-tricalcique (α-TCP) avec de l'hydroxyapatite (HA) ; dans laquelle la pâte macroporeuse est non frittée et dans laquelle la pâte macroporeuse ne comprend aucun détergent.

14. Pâte macroporeuse selon la revendication 13, telle que définie en outre dans l'une quelconque des revendications 4-8 ou 10-12.

15. Pâte macroporeuse pouvant être obtenue par le procédé selon la revendication 2, tel que défini en outre dans l'une quelconque des revendications 3 à 12.

16. Pâte macroporeuse selon l'une quelconque des revendications 13 à 15 destinée à être utilisée dans un procédé d'ingénierie de tissu osseux, le procédé comprenant les étapes :
(c1) implanter ou injecter la pâte macroporeuse dans un être humain ou un animal;
(c2) stabiliser *in situ* la pâte macroporeuse en une forme souhaitée ;
(d) minéraliser la pâte poreuse mise en forme stabilisée en un échafaudage à base d'hydroxyapatite carencée en calcium (CDHA) macroporeux *in situ ;*
dans lequel le procédé ne comporte aucune étape de frittage à haute température ;
et dans lequel le procédé n'implique pas un ajout de détergents.

17. Pâte macroporeuse selon l'une quelconque des revendications 13 à 15 destinée à être utilisée dans un procédé d'ingénierie de tissu osseux, le procédé comprenant les étapes :
(c2) stabiliser la pâte macroporeuse en une forme souhaitée *in vitro ;*
(c1) implanter la pâte macroporeuse mise en forme stabilisée dans un être humain ou un animal;
(d) minéraliser la pâte poreuse mise en forme stabilisée en un échafaudage à base d'hydroxyapatite carencée en calcium (CDHA) macroporeux *in situ ;*
dans lequel le procédé ne comporte aucune étape de frittage à haute température ;
et dans lequel le procédé n'implique pas un ajout de détergents.

18. Utilisation d'une protéine de soie non dénaturée en tant qu'agent moussant dans une pâte macroporeuse appropriée pour obtenir des échafaudages macroporeux à base d'hydroxyapatite carencée en calcium (CDHA).

19. Utilisation selon la revendication 18, dans laquelle la protéine de soie est une protéine de soie d'araignée.

20. Utilisation selon l'une quelconque des revendications 18 à 19, dans laquelle la protéine de soie contient un motif de liaison cellulaire (CBM) en tant que fragment fonctionnel.
